(19) Europäisches Patentamt
European Patent Office
Office européen des brevets

(11) Veröffentlichungsnummer: **0 019 227**
**B1**

# (12) EUROPÄISCHE PATENTSCHRIFT

(45) Veröffentlichungstag der Patentschrift: **24.03.82**

(51) Int. Cl.³: **C 07 C 121/38**

(21) Anmeldenummer: **80102540.4**

(22) Anmeldetag: **08.05.80**

## (54) Verfahren zur Herstellung von Cyanhydrinacylaten von Aldehyden.

(30) Priorität: **17.05.79 DE 2919974**

(43) Veröffentlichungstag der Anmeldung: **26.11.80 Patentblatt 80/24**

(45) Bekanntmachung des Hinweises auf die Patenterteilung: **24.03.82 Patentblatt 82/12**

(84) Benannte Vertragsstaaten: **CH DE FR GB IT LI NL**

(56) Entgegenhaltungen: **DE-C-810 026**

(73) Patentinhaber: **HOECHST AKTIENGESELLSCHAFT, Postfach 80 03 20, D-6230 Frankfurt/Main 80 (DE)**

(72) Erfinder: **Mündnich, Rainer, Dr., Schreyerstrasse 11, D-6000 Frankfurt am Main 70 (DE)**
Erfinder: **Finke, Manfred, Dr., Behringstrasse 25, D-6233 Kelkheim (Taunus) (DE)**
Erfinder: **Rupp, Walter, Dr., Am Eichkopf 6, D-6240 Königstein/Taunus (DE)**
Erfinder: **Dehmer, Klaus, Dr., Falkensteiner Strasse 20, D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 227 B1

# Verfahren zur Herstellung von Cyanhydrinacylaten von Aldehyden

Cyanhydrinacylate von Aldehyden sind wertvolle Zwischenprodukte auf verschiedenen Sachgebieten, insbesondere auf dem Pharma- und dem Pflanzenschutzgebiet.

Zur Herstellung von Cyanhydrinacylaten von Aldehyden ist eine Reihe verschiedener Verfahren bekannt. Die wichtigsten Verfahren können im wesentlichen in zwei Gruppen eingeteilt werden, nämlich in

2-Stufen- und
1-Stufen-Verfahren.

Bei den 2-Stufen-Verfahren wird in der ersten Stufe aus dem jeweiligen Aldehyd und HCN bzw. wässrigem Cyanid zunächst das entsprechende Aldehyd-Cyanhydrin hergestellt, welches dann in der zweiten Verfahrensstufe durch Reaktion mit einem organischen Säurehalogenid oder -anhydrid in das gewünschte Aldehyd-Cyanhydrinacylat übergeführt wird.

Nach einem derartigen – allerdings bereits älteren – 2-Stufen-Verfahren kann z.B. das Acroleincyanhydrinacetat – das wohl bekannteste Cyanhydrinacylat eines α,-β-ungesättigten Aldehyds – hergestellt werden [Van Sleen, Rec. Trav. Chem. P.-B. 21, S. 209 ff, insbesondere S. 215 (1902)]. Demnach wird das zunächst auf übliche Weise aus Acrolein und HCN bzw. wässrigem Cyanid erhaltene Acroleincyanhydrin mit überschüssigem Acetanhydrid in Gegenwart von etwa 50 Mol-% Natriumacetat bei Temperaturen bis zu etwa 80 °C umgesetzt; aus den mitgeteilten Zahlenwerten errechnet sich für die Umsetzung des Acroleincyanhydrins mit dem Acetanhydrid eine Ausbeute von etwa 33% d.Th.

R. Rambaud [Bull. Soc. Chim. 1, S. 1317 ff., insbes. S. 1326 (1934)] gibt an, nach derselben Methode eine Ausbeute von 70% d.Th. an Acroleincyanhydrinacetat erhalten zu haben.

Die ziemlich aufwendige Aufarbeitung des Reaktionsgemisches (s. Van Sleen, a.a.O.) lässt die Methode jedoch für die technische Durchführung wenig geeignet erscheinen; denn das Acroleincyanhydrinacetat muss aus dem Reaktionsgemisch nach der Zersetzung des überschüssigen Acetanhydrids – das daher nicht in den Prozess zurückgeführt werden kann – mit Äther extrahiert, die erhaltene organische Phase mit Natriumcarbonatlösung neutralisiert, über wasserfreiem Natriumsulfat getrocknet, filtriert, eingeengt und schliesslich durch Vakuumdestillation weiter gereinigt werden.

Ausserdem besitzt das so hergestellte Acroleincyanhydrinacetat – wie eigene Versuche ergeben haben – eine (gaschromatographisch bestimmte) Reinheit von bestenfalls nur etwa 83%, so dass wohl auch Rambaud kaum mehr als etwa 58% d.Th. an reinem Acroleincyanhydrinacetat erhalten haben kann. Wegen der ungenügenden Reinheit des bei diesem bekannten Verfahren anfallenden Produkts kann dieses für eine Reihe von Umsetzungen, wie z.B. für Radikalreaktionen, bei denen schon geringfügige Verunreinigungen erheblich stören, nicht ohne eine zusätzliche Reinigung verwendet werden.

Eine neuere Methode zur Herstellung von Acetaldehyd-cyanhydrinacetat [R.M. Nowak, J. Org. Chem. 28, S. 1182-1187, insbes. S. 1186 (1963)] geht aus von einer wässrigen NaCN (2 Mol)-Lösung, welcher zunächst langsam Acetaldehyd (1 Mol) zugesetzt wird. Bei einer Temperatur zwischen −10 °C und 0 °C wird dann Acetylchlorid (1,2 Mol) oder Acetanhydrid zugetropft und die später noch mit Wasser verdünnte Lösung mit Äther extrahiert und die Ätherschicht fraktioniert.

Die dabei erzielte Ausbeute an Acetaldehydcyanhydrinacetat wird zu 90% d.Th. angegeben. Weiterhin wird ausdrücklich betont, dass für optimale Ausbeuten ein NaCN-Überschuss erforderlich ist – im Beispiel beträgt der Überschuss 100% – und äquimolare Mengen an Cyanid und Aldehyd die Ausbeute auf etwa 55–65% verringern.

Nach der einstufigen Verfahrensweise gemäss DE-PS 810 026 soll die Umsetzung von Aldehyden oder Ketonen mit Cyanwasserstoff und acylierend wirkenden Verbindungen wie z.B. Essigsäureanhydrid im Molverhältnis 1:(1–1,2):1 hohe Ausbeuten an den entsprechenden Cyanhydrinacylaten liefern; für dieses Verfahren ist jedoch das Arbeiten mit dem ausserordentlich unangenehm handzuhabenden freien Cyanwasserstoff nachteilig.

Eine weitere Ausführungsart der einstufigen Verfahrensweise zur Herstellung von Cyanhydrinacylaten von Aldehyden wurde z.B. beschrieben von R. Palm, H. Ohse und H. Cherdron in Angew. Chem. 78, S. 1093 (1966) anhand der Herstellung des Acroleincyanhydrinacetats. Demnach wurde zu einer gekühlten Mischung von Acrolein (0,77 Mol) in Benzol Essigsäureanyhdrid (0,77 Mol) rasch zugetropft – wobei praktisch noch keine Reaktion eintritt – und anschliessend eine Lösung von NaCN (1,12 Mol) in Wasser zugefügt. Unter Kühlung (−10 °C) wurde noch einige Zeit nachgerührt, auf 0 °C erwärmen lassen und der Ansatz auf übliche Weise (Neutralisation und Trocknung der organischen Phase vor der Destillation) aufgearbeitet. Die erhaltene Ausbeute an Acroleincyanhydrinacetat soll 87% d.Th. betragen haben. Es ist bemerkenswert, dass auch bei diesem Verfahren ein erheblicher Cyanid-Überschuss (etwa 45%) gegenüber der theoretisch notwendigen Menge verwendet wurde und eine relativ aufwendige Aufarbeitung notwendig war.

Bei einem weiteren bekannten 1-Stufen-Verfahren zur Herstellung u.a. von Cyanhydrinacylaten von Aldehyden (McIntosh, Can. J. Chem. 55, S. 4200 ff., insbesondere S. 4204 (1977)) wird zwar ein geringerer Cyanid-Überschuss (20%) angewandt; dafür wird hier aber noch ein Phasentransferkatalysator (Triäthylbenzylammoniumchlorid) zugesetzt. Bei dem Verfahren wird einer Mischung von KCN, Wasser, Methylenchlorid und dem Phasentransferkatalysator eine Mischung von Aldehyd und Acetanhydrid in Methylenchlorid bei 0 °C zugetropft. Im Falle etwa der Herstellung des

Crotonaldehyd-cyanhydrinacetats soll die Ausbeute 66% d.Th. an isoliertem Produkt betragen haben.

Die bekannten Methoden zur Herstellung von Cyanhydrinacylaten von Aldehyden sind insbesondere für die Durchführung in technischem Massstab nur unzureichend geeignet, denn entweder liefern diese Methoden nur eine ungenügende Ausbeute und Reinheit des gewünschten Produkts (VanSleen, Rambaud, a.a.O.) oder sie erfordern ein Arbeiten mit dem ausserordentlich unangenehm handzuhabenden freien Cyanwasserstoff (DE-PS 810 026) oder es sind – wenn nicht mit freiem Cyanwasserstoff bzw. freier Blausäure, sondern mit deren Salzen (Cyanide) gearbeitet wird – teilweise hohe Überschüsse an Acylierungsmitteln und insbesondere Cyanid nötig (Nowak; Palm, Ohse und Cherdron, a.a.O.), was nicht nur eine aufwendige Isolierung und Produktreinigung erfordert, sondern auch noch eine erhebliche Abwasserreinigung bedingt. Oder es muss – wenn nur ein geringerer Cyanidüberschuss angewandt wird (McIntosh, a.a.O.) – noch ein Phasentransferkatalysator eingesetzt werden, dessen Abtrennung und Rückgewinnung ebenfalls einen erheblichen Aufwand bedeuten.

Es bestand daher die Aufgabe, ein verbessertes Verfahren zur Herstellung von Cyanhydrinacylaten von Aldehyden zu finden, welches die Nachteile der bekannten Verfahren nicht mehr aufweist und auch in technischem Massstab gut und wirtschaftlich durchführbar ist.

Diese Aufgabe konnte erfindungsgemäss dadurch in einfacher und befriedigender Weise gelöst werden, dass bei den an sich bekannten von Aldehyden, Cyanid und Acylierungsmitteln ausgehenden 1-Stufen-Verfahren praktisch keine Überschüsse an Cyanid- und Säurechlorid oder -anhydrid mehr Verwendung finden.

Erfindungsgegenstand ist somit ein Verfahren zur Herstellung von Cyanhydrinacylaten von Aldehyden durch Umsetzung von Aldehyden mit wässrigen Lösungen von Cyaniden und organischen Säurechloriden oder -anhydriden, gegebenenfalls in Gegenwart eines inerten, organischen, mit Wasser nicht mischbaren Lösungsmittels, im 1-Stufen-Verfahren, das dadurch gekennzeichnet ist, dass man die Reaktionskomponenten Aldehyd, Cyanid und Säurechlorid oder -anhydrid im Molverhältnis von etwa 1:(1–1,1):(1–1,1) verwendet. Es war überraschend, dass diese Verfahrensweise gute bis sehr gute Ausbeuten an den gewünschten Produkten liefert, weil man davon ausgehen musste, dass das bei der Umsetzung mit freiem Cyanwasserstoff verwandte Molverhältnis von Aldehyd/(oder Keton), Cyanwasserstoff und Acylierungsmittel = 1:(1–1,2):1 (DE-PS 810 026) sich nicht auch auf das Arbeiten mit Cyaniden (anstelle des freien Cyanwasserstoffs) übertragen lässt; denn von dem mit Cyaniden arbeitenden Stand der Technik her war es bekannt, dass dieses Verfahren nur mit teilweise beträchtlichen Überschüssen an Cyanid (Nowak; Palm, Ohse und Cherdron; a.a.O.) oder – bei geringerem Cyanidüberschuss – nur in Gegenwart von bestimmten Phasentransferkatalysatoren (McIntosh, a.a.O.) in befriedigenden Ausbeuten abläuft.

Das erfindungsgemässe Verfahren kann im Prinzip auf alle möglichen Aldehyde angewandt werden; vorzugsweise werden als Ausgangs-Aldehyde jedoch Verbindungen der Formel I

$R^1$–CHO (I)

worin $R^1$ = ggf. subst.
($C_1$–$C_8$)-Alkyl (verzweigt oder unverzweigt),
($C_3$–$C_6$)-Cycloalkyl,
($C_2$–$C_6$)-Alkenyl (verzweigt oder unverzweigt),
($C_6$–$C_{10}$)–Aryl oder
Benzyl, vorzugsweise
($C_1$–$C_4$)–Alkyl,
($C_2$–$C_3$)– Alkenyl,
insbesondere Vinyl verwendet.

Wenn die Gruppen $R^1$ noch substituiert sind, kommen als Substituenten im Prinzip alle möglichen unter den Reaktionsbedingungen inerten Substituenten in Frage. Im Falle der Substitution von $R^1$ sind vorzugsweise die Alkyl- und Alkenylgruppen substituiert. Bevorzugte Substituenten sind:

Halogen,
($C_1$–$C_4$)-Alkoxy,
Phenyl,
Benzyl,
[($C_1$–$C_4$)-Alkoxy]-carbonyl,

Als Aldehyde I sind in beispielhafter Weise zu nennen: Acetaldehyd, Propional, n-Butanal, i-Butanal, Hexanal, Chloracetaldehyd, Diäthoxyacetaldehyd, Malondialdehydmonodimethylacetal, Succindialdehydmonodiäthylacetal,

Glyoxylsäuremethylester,
Cyclohexylaldehyd,
Acrolein, Methacrolein, Äthacrolein, Crotonaldehyd, Zimtaldehyd,
Benzaldehyd,
Phenylacetaldehyd etc.

Als Cyanide zum Einsatz beim erfindungsgemässen Verfahren sind in erster Linie Alkali- und Ammoniumcyanide, insbesondere Na-, K- und Ammonium-cynaid zu nennen. Die Cyanide besitzen die allgemeine Formel II

MCN (II),

worin M = Alkalimetall- oder Ammoniumion, vorzugsweise Na, K- oder $NH_4$-ion.

Die Cyanide werden zweckmässig in wässriger Lösung – wie auch bei den bekannten Verfahren – angewandt.

Als organische Säurechloride und -anhydride kommen für das erfindungsgemässe Verfahren praktisch alle möglichen aliphatischen und aromatischen Säurechloride und -anhydride in Frage; vorzugsweise kommen Verbindungen der Formel III zum Einsatz:

$$\begin{matrix} O \\ \| \\ R^2 - C - R^3 \end{matrix} \qquad (III),$$

worin $R^2$ = $(C_1$–$C_4)$-Alkyl oder Phenyl, vorzugsweise $CH_3$ oder $C_2H_5$, insbesondere $CH_3$, und $R^3$ = Cl, Br oder

$$\begin{matrix} O \\ \| \\ -O - C - R^2 \end{matrix}$$

vorzugsweise Cl oder $-O-\overset{\overset{\displaystyle O}{\|}}{C}-R^2$.

An konkreten derartigen organischen Säurechloriden und -anhydriden sind in beispielhafter Weise zu nennen:

Acetylchlorid, Propionylchlorid, Buttersäurechlorid,
Acetylbromid,
Benzoylchlorid,
Acetanhydrid, Propionsäureanhydrid, Buttersäureanhydrid etc.

Bevorzugte Säurechloride und -anhydride sind Acetylchlorid und Propionylchlorid sowie die entsprechenden Anhydride, vor allem Acetylchlorid und -anhydrid.

Die organischen Säurechloride und -anhydride können – ebenso wie auch die Ausgangs-Aldehyde – sowohl als solche als auch gelöst in einem inerten organischen, mit Wasser nicht mischbaren Lösungsmittel wie z.B. Methylenchlorid, Tetrachlorkohlenstoff, Benzol, Toluol, Äther etc. eingesetzt werden. Für das Verfahren ist die Einhaltung eines praktisch stöchiometrischen Molverhältnisses von Aldehyd zu Cyanid und Säurechlorid oder -anhydrid wesentlich. Erheblich höhere als etwa 10%ige Abweichungen von dem Molverhältnis 1:1:1 werden nicht angewandt; sie beeinträchtigen vor allem die Wirtschaftlichkeit des Verfahrens, insbesondere da grosse Mengen cyanidhaltige Abwässer anfallen.

Weiterhin ist für den günstigen und wirtschaftlichen Verlauf des Verfahrens die Abwesenheit von Phasentransferkatalysatoren – wie sie etwa bei der von McIntosh a.a.O. beschriebenen Verfahrensweise verwendet werden – bevorzugt.

Das erfindungsgemässe Verfahren kann sowohl diskontinuierlich als auch kontinuierlich durchgeführt werden.

Die diskontinuierliche Durchführung kann im Prinzip in der gleichen Weise erfolgen, wie dies bei den bekannten einschlägigen 1-Stufen-Verfahren des Standes der Technik zur Herstellung von Cyanhydrinacylaten von Aldehyden geschieht – nur dass eben im vorliegenden Fall ein anderes Molverhältnis der Reaktionspartner (etwa stöchiometrisch) und vorzugsweise keine Phasentransferkatalysatoren verwendet werden. So kann man etwa den Aldehyd alleine oder zusammen mit dem organischen Säurechlorid oder -anhydrid, gegebenenfalls gelöst in einem inerten organischen Lösungsmittel, vorlegen und dazu unter Kühlung die wässrige Cyanidlösung zutropfen lassen. Oder man kann die wässrige Cyanidlösung vorlegen und unter Kühlung den Aldehyd und das Säurechlorid oder -anhydrid, gegebenenfalls in Lösung in einem inerten organischen Lösungsmittel, unter Kühlung zugeben, was eine bevorzugte Ausführungsart ist. Auch etwa die gleichzeitige Eindosierung aller 3 Komponenten in das Reaktionsgefäss ist möglich. Als Reaktionstemperaturen für die diskontinuierliche Verfahrensweise sind Temperaturen zwischen etwa −20 und +20 °C, vorzugsweise zwischen etwa −10 und +5 °C zweckmässig.

Die entstehende 2phasige Reaktionsmischung wird in organische und wässrige Phase getrennt, die wässrige Phase gegebenenfalls noch mit einem inerten organischen Lösungsmittel extrahiert und der Extrakt zusammen mit der vorher abgetrennten organischen Phase wie üblich aufgearbeitet (destilliert).

Bevorzugt ist jedoch die kontinuierliche Durchführung des Verfahrens. Dabei werden laufend gleichzeitig alle drei Reaktionskomponenten in den Reaktor eindosiert und die 2phasige Reaktionslösung wird kontinuierlich ausgekreist sowie einem Phasentrenner zugeführt. Die organische Phase wird dann wie beim diskontinuierlichen Verfahren aufgearbeitet. Selbstverständlich können auch bei der kontinuierlichen Verfahrensweise inerte organische Lösungsmittel mitverwendet und der Aldehyd und das Acylierungsmittel, die zumindest bei nicht erhöhten Temperaturen kaum miteinander reagieren, gegebenenfalls vorgemischt werden. Für die kontinuierliche Verfahrensweise kommen Reaktionstemperaturen zwischen etwa −20 und 70 °C, vorzugsweise zwischen etwa 0 und 40 °C in Frage.

Ein Schutzgas wie z.B. Stickstoff oder Argon ist für das Gelingen der Reaktion nicht erforderlich, aber auch nicht schädlich. Die Cyanhydrinacylate fallen meist flüssig an und können deshalb durch Destillation gereinigt werden.

Falls bei der Reaktion ein organisches Lösungsmittel verwendet wurde, wird es bei der (destillativen) Aufarbeitung der Reaktionsmischung zurückgewonnen und kann daher wieder verwendet werden.

Im bei dem Verfahren anfallenden Abwasser können gegebenenfalls vorhandene geringe Cyanidmengen durch eine der bekannten Methoden (z.B. $H_2O_2$ oder Formalin) unschädlich gemacht werden.

Die mit Hilfe des erfindungsgemässen Verfahrens erzielten Ausbeuten an Aldehyd-cyanhydrinacylaten betragen durchweg zwischen etwa 80 und 95% d.Th., bezogen auf den Ausgangs-Aldehyd. Wenn man als Ausgangs-Aldehyde Verbindungen der Formel I und als organische Säurechloride oder -anhydride Verbindungen der Formel III verwendet, besitzen die Endprodukte die Formel IV

```
R¹ – CH– CN                (IV)
      |
      O – C – R²
          ||
          O
```

worin $R^1$ und $R^2$ die bei den Formeln I und III beschriebene Bedeutung besitzen.

Wegen der guten bis sehr guten Ausbeute, der einfachen Aufarbeitung (Destillation der organischen Phase, ohne vorherige Neutralisation und Trocknung) und hohen Reinheit der anfallenden Produkte sowie der Tatsache, dass infolge der praktisch stöchiometrischen Mengen der Reaktionspartner kaum unverbrauchte Komponenten übrig bleiben und etwa wieder aufgearbeitet oder beseitigt werden müssen (mit Ausnahme höchstens geringer Cyanidmengen, die aber auf einfache Weise unschädlich gemacht werden können) stellt das Verfahren einen erheblichen Fortschritt dar. Bei der kontinuierlichen Durchführungsweise fällt für den Fortschritt noch zusätzlich ins Gewicht, dass hier bei höheren Temperaturen gearbeitet werden kann, wodurch die Einsparung von teilweise erheblichem Kühlungsaufwand möglich ist.

Beispiel 1
Acetaldehydcyanhydrinacetat

In einem Kolben werden 400 g 27%iger wässriger NaCN-Lösung (2,2 Mol) und 150 ml Methylenchlorid vorgelegt. Getrennt aber gleichzeitig werden 88 g Acetaldehyd (2,0 Mol) vermischt mit 138 ml Methylenchlorid und 224 g Acetanhydrid (2,2 Mol) vermischt mit 42 ml Methylenchlorid unter Kühlung und Rühren bei −10 °C aus gekühlten Tropftrichtern zugetropft. Nach beendetem Zutropfen und Zugabe von 150 ml Wasser werden die Phasen getrennt. Die organische Phase wird zuerst bei Normaldruck und dann im Wasserstrahlvakuum fraktioniert. Man erhält bei 20 mbar und 81 ° C 204,6 g Acetaldehydcyanhydrinacetat (90,5% d.Th.).

Beispiel 2
Isobutyraldehydcyanhydrinacetat

In einem Kolben werden 600 g einer 27%igen wässrigen NaCN-Lsg. (3,3 Mol) vorgelegt und gleichzeitig aber getrennt 217 g iso-Butyraldehyd (3,0 Mol) und 337 g Acetanhydrid (3,3 Mol) während 1 Stunde bei 0—10 °C zugetropft. Nach Zugeben von 250 ml Wasser und Trennung der Phasen wird die organische Phase am Wasserstrahlvakuum destilliert.
$Kp_{20\ mbar}$ 78 °C. Ausbeute 344,5 g (81,5% d.Th.)

Beispiele 3–8: Acroleincyanhydrinacetat

Beispiel 3

400 g einer 27%igen wässrigen NaCN-Lsg. (2,2 Mol) werden in einem Kolben vorgelegt und auf 0 °C gekühlt. Dazu werden 118 g 95%iges Acrolein (2,0 Mol) und 224,5 g Acetanhydrid (2,2 Mol), die man vorher vermischt hat, während 50 Minuten unter Rühren zugetropft. Die Reaktionstemperatur wird durch äussere Kühlung bei 0 °C gehalten. Nach beendigtem Eintropfen werden zur Lösung ausgefallener anorganischer Salze 150 ml Wasser der Reaktionsmischung zugesetzt. Nach dem Abtrennen des Produktes im Scheidetrichter wird am Wasserstrahlvakuum destilliert. Man erhält bei einem $Kp_{20\ mbar}$ 73 °C 235 g Acroleincyanhydrinacetat (94% d.Th.).
Reinheit (GC): 97,1%

Beispiel 4

In einem 4 Ltr.-Kolben werden 814 g 26,5%ige wässrige NaCN-Lsg. (4,4 Mol) und 800 g Methylenchlorid vorgelegt und auf 0° gekühlt. Unter Rühren werden 236 g 95%iges Acrolein (4,0 Mol) vermischt mit 180 g Methylenchlorid sowie 449 g Acetanhydrid (4,4 Mol) getrennt aber gleichzeitig während 30 Minuten zugetropft. Die Innentemperatur wird dabei durch äussere Kühlung auf 0 °C gehalten. Nach beendetem Zutropfen werden 360 g Wasser zur Lösung ausgefallener Salze zugesetzt und die Phasen getrennt. Die untere organische Phase wird ohne Trocknung destilliert. Nach dem Abdestillieren des Methylenchlorids unter Normaldruck, destillieren bei 20 mbar und 73 °C 480 g Acroleincyanhydrinacetat (96% d.Th.) Reinheit (GC): 98%ig.

Beispiel 5

In einem mit Wasser gefüllten 250 ml-Reaktor mit Überlauf werden unter Rühren über Steigrohre gleichzeitig aber getrennt 1220 g 26,5%ige wässrige NaCN-Lsg. (6,6 Mol ), 354 g 95%iges Acrolein vermischt mit 472 g Methylenchlorid und 673 g Acetanhydrid (6,6 Mol) vermischt mit 320 g Methylenchlorid während 1 Stunde zugetropft. Durch äussere Kühlung wird die Innentemperatur des Reaktors auf 18–20 °C gehalten. Die aus dem Reaktor kontinuierlich ausfliessende zweiphasige Reaktionslösung wird einem Phasentrenner zugeführt und die untere organische Phase abgetrennt und einer Destillation zugeführt. Nach dem Abdestillieren des Methylenchlorids unter Normaldruck, destillieren bei 21 mbar und 74 °C 660 g Acroleincyanhydrinacetat (88% d.Th.).

Beispiel 5a

Ausführung des Versuches wie unter Beispiel 5 beschrieben, aber bei einer Reaktionstemperatur von 30°.
Ausbeute: 636,6 g Acroleincyanhydrinacetat (≙84,7% d.Th.)

Beispiel 6

In einem Rührkolben werden 551 g 26%ige wässrige KCN-Lösung (2,2 Mol) und 350 ml Methylenchlorid vorgelegt. Dazu werden unter Rühren und äusserer Kühlung bei −10 °C aus gekühlten Tropftrichtern gleichzeitig aber getrennt 118 g 85%iges Acrolein (2,0 Mol) und 157 g Acetylchlorid (2,0 Mol) während 20 Minuten zugetropft. Danach werden 180 ml Wasser zugegeben und die Phasen getrennt. Die wässrige Phase wird mit 100 ml $CH_2Cl_2$ extrahiert und die Methylenchloridphase mit der vorher abgetrennten organischen Phase vereinigt. Nach dem Abdestillieren des Methylenchlorids unter Normaldruck wird Wasserstrahlvakuum angelegt. Es werden 214,4 g Acroleincyanhydrinacetat erhalten (85,5% d.Th.)

Beispiel 7

In einem gekühlten Kolben werden 90 ml Wasser und 17 g einer 27%igen wässrigen NaCN-Lsg. vorgelegt und auf 0 °C gekühlt. Dann werden unter äusserer Kühlung bei dieser Temperatur getrennt aber gleichzeitig 59 g 95%iges (1 Mol) Acrolein vermischt mit 105 g (1,03 Mol) Acetanhydrid und 170 g einer 27%igen wässrigen NaCN-Lsg. (insgesamt 1,03 Mol) zugetropft. Nach beendetem Eintropfen und Zugabe von 80 ml Wasser werden die Phasen getrennt und die organische Phase am Wasserstrahlvakuum destilliert.
Ausbeute: 109,4 g (≙87,5% d.Th.)

Beispiel 8

In ein Zweiphasensystem aus 70 g (1,07 Mol) Kaliumcyanid, 150 ml Wasser und 300 ml Methylenchlorid werden bei − 10 °C unter heftigem Rühren ein auf − 10 °C vorgekühltes Gemisch aus 56 g (1 Mol) Acrolein und 110 g (1,08 Mol) Acetanhydrid in ca. 1h eingetropft. Man lässt das Reaktionsgemisch auf Raumtemperatur kommen, trennt die organische Phase ab und schüttelt die wässrige Phase zweimal mit Methylenchlorid aus. Die organischen Phasen werden vereinigt und vom Lösungsmittel befreit. Anschliessend wird der Rückstand unter vermindertem Druck destilliert.
115 g (92% d.Th.)
$Kp_{26\ mbar}$: 83–85 °C

Beispiel 9

Crotonaldehydcyanhydrinacetat

In einem Kolben werden 400 g einer wässrigen 27%igen NaCN-Lsg. (2,2 Mol) mit 300 ml Methylenchlorid vorgelegt. Unter Rühren und Kühlung werden getrennt aber gleichzeitig 174 g Crotonaldehyd (2,0 Mol) in 145 ml Methylenchlorid und 224 g Acetanhydrid (2,2 Mol) während 35 Minuten bei ca. − 10 °C zugetropft. Nach Zugabe von 150 ml Wasser und Phasentrennung wird die organische Phase zuerst unter Normaldruck und dann unter Wasserstrahlvakuum destilliert. Man erhält bei 25 mbar und 94 °C 243 g Crotonaldehydcyanhydrinacetat (87,5% d.Th.).

Beispiel 10:

Acroleincyanhydrinpropionat

In einem 1 l-Kolben werden 200 g einer wässrigen 27%igen NaCN-Lösung (1,1 Mol) mit 100 ml Methylenchlorid vorgelegt und auf − 15 ° gekühlt. Dazu wird unter Rühren binnen 45 Minuten aus einem gekühlten Tropftrichter eine kurz zuvor bereitete Mischung aus 56 g Acrolein (1,0 Mol), 102 g Propionylchlorid (1,1 Mol) und 200 ml Methylenchlorid getropft. Durch äussere Kühlung wird der Kolbeninhalt auf − 10 bis − 15° gehalten. Nach beendetem Eintropfen werden 70 ml Wasser zugegeben und die Phasen getrennt. Die organische Phase wird zuerst unter Normaldruck und dann im Wasserstrahlvakuum fraktioniert. Man erhält bei 25 mbar und 93–96 °C 116,1 g Acroleincyanhydrinpropionat (83,6% d.Th.).

Beispiel 11

Methacroleincyanhydrinacetat

In einem 1 l-Kolben werden 200 g einer wässrigen 27% igen NaCN-Lösung (1,1 Mol) vorgelegt und auf 0° gekühlt. Dazu wird unter Rühren eine Mischung aus 70 g (1 Mol) Methacrolein und 112,2 g (1,1 Mol) Acetanhydrid binnen 25 Minuten zugetropft. Der Kolbeninhalt wird durch äussere Kühlung auf 0 °C gehalten. Nach beendeter Zugabe werden die ausgefallenen Salze durch Zugabe von 70 ml Wasser gelöst und die Phasen getrennt. Die organische Phase wird im Wasserstrahlvakuum fraktioniert. Man erhält bei 25 mbar und 90–92 °C 118,7 g Methacroleincyanhydrinacetat (85,5% d.Th.).

**Patentansprüche**

1. Verfahren zur Herstellung von Cyanhydrinacylaten von Aldehyden durch Umsetzung von Aldehyden mit wässrigen Lösungen von Cyaniden und organischen Säurechloriden oder -anhydriden, gegebenenfalls in Gegenwart eines inerten organischen, mit Wasser nicht mischbaren Lösungsmittels, im 1-Stufen-Verfahren, dadurch gekennzeichnet, dass man die Reaktionskomponenten Aldehyd, Cyanid und Säurechlorid oder -anhydrid im Molverhältnis von 1:(1–1,1):(1–1,1) verwendet.

2. Verfahren nach Anspruch 1, dadurch gekennzeichnet, dass man als Aldehyde Verbindungen der Formel I

$$R^1-CHO \qquad (I)$$

worin
$R^1$ = gegebenenfalls substituiertes
$(C_1–C_8)$-Alkyl
$(C_3–C_6)$-Cycloalkyl,
$(C_2–C_6)$-Alkenyl
$(C_6–C_{10})$-Aryl oder Benzyl, vorzugsweise
$(C_1–C_4)$-Alkyl, oder
$(C_2–C_3)$ Alkenyl, insbesondere Vinyl verwendet.

3. Verfahren nach den Ansprüchen 1–2, dadurch gekennzeichnet, dass man als Cyanide Verbindungen der Formel II

$$MCN \qquad (II)$$

worin M = Alkalimetall- oder Ammoniumion, vorzugsweise Na-, K- oder $NH_4$-Ion verwendet.

4. Verfahren nach den Ansprüchen 1–3, dadurch gekennzeichnet, dass man als Säurechloride oder -anhydride Verbindungen der Formel III

$$R^2-\overset{\overset{\large O}{\|}}{C}-R^3 \qquad (III),$$

worin $R^2$ = $(C_1–C_4)$-Alkyl oder Phenyl, vorzugsweise $CH_3$ oder $C_2H_5$, insbesondere $CH_3$, und

O
||
$R^3$ = Cl, Br oder – O – C – $R^2$,

O
||
vorzugsweise Cl oder –O–C–$R^2$,

verwendet.

5. Verfahren nach den Ansprüchen 1–4, dadurch gekennzeichnet, dass man die Umsetzung in Abwesenheit von Phasentransferkatalysatoren durchgeführt.

6. Verfahren nach den Ansprüchen 1–5, dadurch gekennzeichnet, dass man die Umsetzung kontinuierlich durch gleichzeitiges Eindosieren der Reaktionskomponenten in das Reaktionsgefäss durchführt.

7. Verfahren nach Anspruch 6, dadurch gekennzeichnet, dass man die kontinuierliche Umsetzung bei Temperaturen zwischen 0 und 40 °C durchführt.

**Revendications**

1. Procédé de préparation d'acylates de cyanhydrines d'aldéhydes par réaction d'aldéhydes avec des solutions aqueuses de cyanures et des chlorures ou anhydrides d'acides organiques, éventuellement en présence d'un solvant organique inerte non miscible à l'eau, par un mode opératoire à une seule étape, procédé caractérisé en ce qu'on utilise les composantes réactionnelles, c'est-à-dire l'aldéhyde, le cyanure et le chlorure ou l'anhydride d'acide, dans une proportion molaire de 1:(1–1,1):(1–1,1).

2. Procédé selon la revendication 1, caractérisé en ce qu'on utilise, comme aldéhydes, des composés répondant à la formule I

$R^1$ – CHO (I)

dans laquelle $R^1$ représente un radical alkyle en $C_1$–$C_8$, linéaire ou ramifié, un radical cycloalkyle en $C_3$–$C_6$, un radical alcényle en $C_2$–$C_6$, linéaire ou ramifié, un radical aryle en $C_6$–$C_{10}$ ou un radical benzyle, chacun de ces radicaux étant éventuellement substitué, de préférence un radical alkyle en $C_1$–$C_4$ ou un radical alcényle en $C_2$ ou $C_3$, plus spécialement le radical vinyle.

3. Procédé selon l'une des revendications 1 et 2, caractérisé en ce qu'on utilise, comme cyanures, des composés répondant à la formule II

MCN (II)

dans laquelle M représente un ion de métal alcalin ou d'ammonium, de préférence un ion Na, K ou $NH_4$.

4. Procédé selon l'une des revendications 1 à 3, caractérisé en ce qu'on utilise, comme chlorures ou anhydrides d'acides, des composés répondant à la formule III

O
||
$R^2$ – C– $R^3$ (III)

dans laquelle $R^2$ représente un radical alkyle en $C_1$–$C_4$ ou un radical phényle, de préférence $CH_3$ ou $C_2H_5$, plus spécialement $CH_3$, et $R^3$ représente Cl, Br ou

O
||
– O – C– $R^2$,

O
||
de préférence Cl ou – O – C – $R^2$.

5. Procédé selon l'une quelconque des revendications 1 à 4, caractérisé en ce qu'on effectue la réaction en l'absence de catalyseurs de transfert de phase.

6. Procédé selon l'une quelconque des revendications 1 à 5, caractérisé en ce qu'on effectue la réaction en continu en introduisant progressivement et simultanément les composantes réactionnelles dans le récipient réactionnel.

7. Procédé selon la revendication 6, caractérisé en ce que la réaction en continu est effectuée à des températures comprises entre 0 et 40 °C.

**Claims**

1. Process for the manufacture of cyanohydrin acylates of aldehydes by reacting aldehydes with aqueous solutions of cyanides and organic acid chlorides or acid anhydrides, optionally in the presence of an inert organic solvent immiscible with water, in a one-stage process, characterized in using the reactants aldehyde, cyanide and acid chloride or anhydride in the molar ratio of 1:(1–1.1):(1–1.1).

2. Process as claimed in claim 1, characterized in using as aldehydes compounds of the formula I

$R^1$ – CHO (I),

in which $R^1$ is an optionally substituted
($C_1$–$C_8$)-alkyl,
($C_3$–$C_6$)-cycloalkyl,
($C_2$–$C_6$)-alkenyl,
($C_6$–$C_{10}$)-aryl or benzyl, preferably
($C_1$–$C_4$)-alkyl, or
($C_2$–$C_3$)-alkenyl, especially vinyl.

3. Process as claimed in claims 1 to 2, characterized in using as cyanides compounds of the formula II

MCN (II),

in which M is an alkali metal ion or ammonium ion, preferably a Na-, K- or $NH_4$-ion.

4. Process as claimed in claims 1 to 3, characterized in using as acid chlorides or anhydrides compounds of the formula III

$$R^2-\overset{\overset{\displaystyle O}{\|}}{C}-R^3 \qquad (III),$$

in which $R^2$ is $(C_1–C_4)$-alkyl or phenyl, preferably $CH_3$ or $C_2H_5$, especially $CH_3$,

and $R^3$ is Cl, Br or $-O-\overset{\overset{\displaystyle O}{\|}}{C}-R^3$,

preferably Cl or $-O-\overset{\overset{\displaystyle O}{\|}}{C}-R^2$.

5. Process as claimed in claims 1 to 4, characterized in carrying out the reaction in the absence of phase transfer catalysts.

6. Process as claimed in claims 1 to 5, characterized in carrying out the reaction continuously by simultaneously introducing the reactants into the reaction vessel.

7. Process as claimed in claim 6, characterized in carrying out the continuous reaction at temperatures of from 0 to 40 °C.